# DEMANDE DE BREVET EUROPEEN

(11) **EP 0 913 391 A1**
(43) Date de publication de la demande: **06.05.1999**
(21) Numéro de dépôt: 98402404.2
(22) Date de dépôt: 30.09.1998
(51) Int. Cl.: C07C 303/44, B01D 61/44

(54) **Dessalement de solutions aqueuses de sulfonamides**

(30) Priorité: 15.10.1997 FR 9712901
(71) Demandeur: ELF ATOCHEM S.A., 92800 Puteaux, Hauts-de-Seine (FR)
(72) Inventeur: Gancet, Christian, 64140 Lons (FR); Lauranson, Didier, 64140 Lons (FR); Perie, Frédéric, 64140 Billere (FR)
(74) Mandataire: Leboulenger, Jean

(57) **Abrégé**

Pour éliminer les sels (en particulier NH₄Cl) présents dans une solution aqueuse de sulfonamide (en particulier CH₃SO₂NH₂), on soumet la solution à une électrodialyse à deux compartiments.

Par maintien du pH à une valeur inférieure à 7, on évite la formation d'ammoniac. La solution déminéralisée peut être concentrée pour récupérer, après cristallisation, le sulfonamide.

## Description

La présente invention concerne le domaine des sulfonamides hydrosolubles et a plus particulièrement pour objet un procédé permettant la récupération et la purification des sulfonamides obtenus par un procédé de synthèse en phase aqueuse. L'invention concerne plus spécialement le méthanesulfonamide CH₃SO₂NH₂ (appelé aussi MSAM), mais plus généralement tous les sulfonamides hydrosolubles.

Lorsque les sulfonamides sont préparés en phase aqueuse, on obtient finalement une solution aqueuse de sulfonamide qui, pour pouvoir isoler le sulfonamide, doit être concentrée de façon à cristalliser le sulfonamide. Malheureusement, la solution aqueuse à concentrer contient généralement des sels, en particulier du chlorure d'ammonium, qui rendent difficile cette étape de concentration.

L'art antérieur ne mentionne pas l'utilisation de technologies membranaires dans les procédés de synthèse de sulfonamides qui, tel celui du brevet FR 2 708 266, font appel à des réactions réalisées en milieu solvant comme l'acétonitrile ou le propionitrile.

L'utilisation de membranes d'électrodialyse pour dessaler des solutions aqueuses est connue de l'homme de l'art et fait notamment l'objet du brevet EP 536 021 qui concerne le dessalage de solutions aqueuses de solvants polaires aprotiques.

L'application de cette technique au dessalage de solutions aqueuses de sulfonamides, issues d'un procédé de synthèse en phase aqueuse, n'est cependant pas connue. Etant donné que les sulfonamides concernés (en particulier le MSAM) ont une masse moléculaire relativement peu élevée, ils sont susceptibles de diffuser de façon importante au travers des membranes et on pouvait craindre de ne pas pouvoir obtenir des rendements de dessalage élevés.

Il a maintenant été trouvé que les sels présents dans une solution aqueuse de sulfonamide peuvent être éliminés avec un rendement de dessalage élevé en soumettant cette solution à une électrodialyse à deux compartiments pourvu que l'on maintienne la solution à un pH acide.

Les sels (en particuliers NH₄Cl) passent de la solution (diluat) contenant le sulfonamide à une solution réceptrice (concentrat) et, dans la mesure où l'on reste à un pH acide pour éviter la diffusion d'ammoniac non protoné, l'élimination de NH₄Cl ne pose pas de problème.

L'invention a donc pour objet un procédé de dessalage d'une solution aqueuse de sulfonamide, caractérisé en ce que cette solution, maintenue à un pH acide, est soumise à une électrodialyse à deux compartiments.

Pour la mise en oeuvre du procédé selon l'invention, on peut utiliser les membranes échangeuses d'ions cationiques et anioniques du commerce, telles que par exemple celles commercialisées par la Société Asahi Glass sous la dénomination SELEMION®, par la Société Tokuyama Soda (dénomination NEOSEPTA®) ou par la Société Aqualytics. Ces membranes commerciales ont en général une épaisseur comprise entre 0,1 et 1 mm et un diamètre de pores compris entre 1 et 30 µm. Les membranes échangeuses d'ions sont habituellement constituées par une matrice polymérique (par exemple polystyrène-divinylbenzène) sur laquelle sont liés chimiquement des groupements anioniques (par exemple carboxylate ou sulfonate) pour les résines échangeuses de cations ou des groupements cationiques (par exemple ammonium substitué) pour les résines échangeuses d'anions. Des membranes dites "sélectives", à structure polymérique plus resserrée, ont été développées pour retenir les ions di- et tri-valents (sulfate, calcium, magnésium,...), et laisser passer les ions monovalents (chlorure, sodium, ..)

La concentration en sulfonamide de la solution à dessaler peut varier dans de larges limites et est généralement comprise entre 0,1 M et la limite de solubilité du sulfonamide considéré. Cette concentration est de préférnce comprise entre 0,5 et 2 M.

Conformément au procédé selon l'invention, la solution à dessaler est maintenue à un pH acide, de préférence entre 2 et 6 et, plus particulièrement, entre 3 et 5. Ceci peut être fait, par exemple, par addition d'HCl dilué (0,05 à 0,5 M) à la solution saline. HCl convient la plupart du temps, mais selon l'anion du sel à éliminer, on peut utiliser d'autres monoacides.

Les exemples suivants illustrent l'invention sans la limiter.

### EXEMPLE 1

La solution à traiter était un mélange équimolaire de méthanesulfonamide (MSAM) et de NH₄Cl à pH 4,4. La solution réceptrice était une solution de chlorure de sodium à 5 g/l, ainsi que la solution d'électrolyte.

On a utilisé un électrodialyseur SRTI de type P1 équipé de membranes standard AMV et CMV de Asahi Glass pour une surface de cellule de 0,138 m² (pour un dispositif à deux compartiments, 1 m² de cellule correspond à 1 m² de membrane cationique + 1 m² de membrane anionique, soit 2 m² de membranes).

La densité de courant imposée était de 435 A/m², sauf en fin d'essai où la conductivité était trop faible ; on a alors imposé une tension de 1,5 V par cellule.

Pendant l'électrodialyse, le pH a été maintenu entre 3 et 5 par addition d'HCl 0,1 M de façon à éviter toute formation d'ammoniac susceptible de diffuser et de contaminer le MSAM produit.

L'analyse du MSAM a été faite par chromatographie gazeuse. Le chlorure d'ammonium a été suivi par conductimétrie en cours d'essai et mesuré par chromatographie ionique sur les échantillons prélevés.

La durée de l'essai a été de 0,66 heure. La productivité du dispositif a été de 11,3 kg de solution 1M en MSAM traitée par heure et par m², l'énergie consommée étant de 45,1 kWh par tonne de solution 1M de MSAM traitée.

Le tableau 1 ci-après indique le niveau de déminéralisation atteint sur la solution de MSAM après le traitement par électrodialyse.

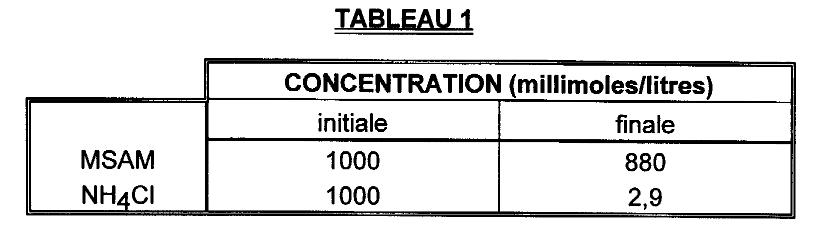

Le tableau 2 suivant indique les concentrations en sel et en MSAM au cours de l'essai, ainsi que le bilan massique pour le MSAM.

**TABLEAU 2**

| | **Compartiment MSAM (diluat)** | **Compartiment sel (concentrat)** |
|---|---|---|
| Concentration en NH₄Cl (millimoles/litre) | | |
| - initiale | 1000 | 85,5 |
| - finale | 2,9 | 1070 |
| Concentration en MSAM (millimoles/litre) | | |
| - initiale | 1000 | 0 |
| - finale | 880 | 55 |
| Bilan massique pour le MSAM (en g) | | |
| - masse initiale | 94 | 0 |
| - masse finale | 83,5 | 7,7 |

Ces résultats indiquent que le MSAM est bien retenu dans le compartiment diluat, les pertes par diffusion passive s'élevant à environ 8,4 % en poids. Le rendement observé est de 91,6 % en poids.

En fonction du niveau de rendement visé en MSAM, la concentration finale en sel peut être ajustée en arrêtant l'électrodialyse avant épuisement de la solution saline.

La concentration finale en NH₄Cl est limitée par la conductivité minimale à conserver pour permettre le passage du courant. Pour obtenir des niveaux de déminéralisation plus poussée, on peut compenser la faible conductivité de la solution en plaçant une résine ou un feutre échangeur d'ions dans le compartiment sel de l'électrodialyseur.

### EXEMPLE 2

Pour traiter un mélange équimolaire de MSAM et de NH₄Cl à pH 4,15, on a opéré comme à l'exemple 1 mais en utilisant des membranes sélectives ASV et CHV de Asahi Glass.

La durée de l'essai a été de 0,7 heure. La productivité du dispositif a été de 10,1 kg de solution 1M en MSAM traitée par heure et par m², l'énergie consommée étant de 45,8 kWh par tonne de solution 1M de MSAM traitée.

Le tableau 3 ci-après indique le niveau de déminéralisation atteint sur la solution de MSAM après le traitement d'électrodialyse.

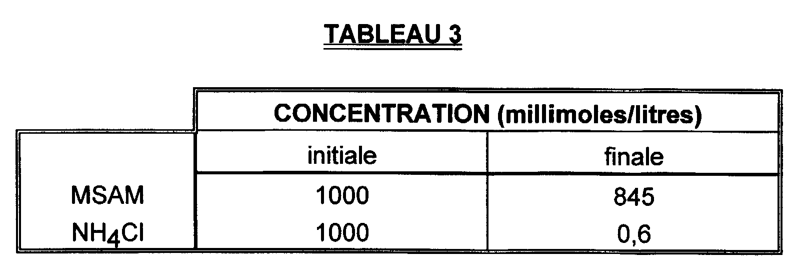

Le tableau 4 suivant indique les concentrations en sel et en MSAM au cours de l'essai, ainsi que le bilan massique pour le MSAM.

**TABLEAU 4**

| | **Compartiment MSAM (diluat)** | **Compartiment sel (concentrat)** |
|---|---|---|
| Concentration en NH₄Cl (millimoles/litre) | | |
| - initiale | 1000 | 93,5 |
| - finale | 0,6 | 835 |
| Concentration en MSAM (millimoles/litre) | | |
| - initiale | 1000 | 0 |
| - finale | 845 | 22 |
| Bilan massique pour le MSAM (en g) | | |
| - masse initiale | 92,5 | 0 |
| - masse finale | 75,2 | 2,6 |

Le MSAM est bien retenu dans le compartiment diluat, les pertes par diffusion passive s'élevant à environ 3,3 % en poids. Le rendement observé est donc de 96,7 % en poids.

Cet exemple montre que l'emploi de membranes plus sélectives (à structure polymérique plus resserrée) permet d'abaisser le niveau de perte en MSAM de 8,4 à 3,3 %.

## Revendications

1. Procédé de dessalage d'une solution aqueuse de sulfonamide, caractérisé en ce que cette solution, maintenue à un pH acide, est soumise à une électrodialyse à deux compartiments

2. Procédé selon la revendication 1, dans lequel le pH est maintenu entre 2 et 6, de préférence entre 3 et 5.

3. Procédé selon la revendication 1 ou 2, dans lequel le maintien du pH acide est effectué par addition d'acide chlorhydrique à la solution saline.

4. Procédé selon l'une des revendications 1 à 3, dans lequel la concentration en sulfonamide de la solution à dessaler est comprise entre 0,1 M et sa limite de solubilité dans la solution saline.

5. Procédé selon la revendication 4, dans lequel la concentration en sulfonamide est comprise entre 0,5 et 2 M.

6. Procédé selon l'une des revendications 1 à 5, dans lequel le sulfonamide est le méthanesulfonamide.
